# FASCICULE DE BREVET EUROPEEN

(11) **EP 0 498 133 B1**
(45) Date de publication et mention de la délivrance du brevet: **06.05.1999**
(21) Numéro de dépôt: 91403570.4
(22) Date de dépôt: 01.01.1992
(51) Int. Cl.: C07K 1/16, C07K 1/18

(54) **Procédé pour isoler de l'albumine humaine**
Verfahren zur Reinigung von menschlichem Albumin
Process for the purification of human albumin

(30) Priorité: 07.02.1991 FR 9101365
(43) Date de publication de la demande: 12.08.1992
(73) Titulaire: PASTEUR MERIEUX SERUMS ET VACCINS, Société Anonyme :, F-69348 Lyon Cédex 07 (FR)
(72) Inventeur: Grandgeorge, Michel Gaston Joseph, F-69670 Vaugneray (FR); Veron, Jean-Luc Bernard, F-69210 Sourcieux-Les-Mines (FR); Fournier, Pierre Luc Jean, F-69005 Lyon (FR)
(74) Mandataire: Bernasconi, Jean

(56) Documents cités:
- EP-A- 0 121 468
- US-A- 4 228 154
- CHEMICAL ABSTRACTS, vol. 112, no. 8, 19 Février 1990, Columbus, Ohio, US; abstract no. 62411, J.F.STOLTZ ET AL.: 'Chromatografic purification of human albumin. Thecnical and economic' page 410 ;colonne R ;

## Description

Procédé pour isoler de l'albumine humaine à partir du surnageant IV, notamment IV-4, ou de la fraction V de COHN ou d'un surnageant ou fraction analogue.

L'invention concerne un procédé pour isoler de l'albumine humaine à partir du surnageant IV, et notamment IV-4, ou de la fraction V de COHN ou d'un surnageant ou fraction plasmatique de composition analogue provenant d'un fractionnement alcoolique ou non, par des étapes de chromatographie échangeuse d'ions.

La demande de brevet français FR-A-2 327 256 décrit la préparation d'albumine à partir d'un plasma dépourvu des immunoglobulines, qui peut être la fraction V de COHN. La purification s'effectue successivement sur un échangeur d'anions hydrophile sur lequel se fixe l'albumine et un échangeur de cations hydrophile. Ce procédé conduit à une albumine de pureté moyenne (> 96 %) et contenant moins de 3,5 % de polymères.

Le brevet US-A-4 228 154 décrit également un procédé de préparation d'albumine, à partir du surnageant II + III de COHN, cette fraction étant débarrassée des immunoglobulines. Le procédé comprend d'abord une filtration sur gel pour éliminer alcool et sels résiduels, puis une adsorption des lipoprotéines sur silice et enfin deux étapes chromatographiques sur un échangeur d'anions hydrophile et un échangeur de cations hydrophile. L'albumine obtenue a une pureté relativement élevée variant entre 96,9 % et 98,9 % en électrophorèse. Dans ce procédé, l'albumine n'est jamais fixée sur le support chromatographique.

Dans la demande de brevet européen EP-A-0 367 220, dans laquelle on part de la fraction V de COHN, il est renoncé à l'étape chromatographique sur échangeur de cations. Le procédé décrit comprend le passage sur une colonne d'échange d'anions hydrophile faible ou fort sur laquelle l'albumine ne se fixe pas et le produit obtenu contient encore des polymères (3 %).

La demande de brevet français FR-A-2 543 448, fondant la priorité de la demande EP-A-121 468, décrit un procédé d'obtention d'une albumine de haute pureté (≥ 99 % en électrophorèse) en partant cette fois-ci d'un plasma brut ou débarrassé du cryoprécipité qui n'a subi qu'une clarification initiale destinée à éliminer les sels et les euglobulines. Le procédé nécessite cependant des étapes de fractionnement chromatographique complexes utilisant au moins un échangeur d'anions et un échangeur de cations, et un support de type hydrophobe, qui peut être l'un des échangeurs d'ions précédents, et, en particulier, un échangeur d'anions hydrophile sur lequel l'albumine se fixe, avant d'être éluée, un échangeur d'anions hydrophobe et un échangeur de cations. L'abrégé n° 62411 de Chemical Abstracts se rapporte à des essais menés sur le procédé objet de la demande EP 121 468. Il divulgue donc également un procédé de purification de l'albumine nécessitant notamment 3 étapes de purification.

La présente invention a pour objectif de fournir un procédé de purification de l'albumine conduisant à une albumine de pureté supérieure à 99 % et exempte de polymères, tout en conservant la sécurité virale apportée par le fractionnement alcoolique de COHN.

La déposante a trouvé de façon surprenante qu'il était possible d'obtenir une telle albumine de pureté électrophorétique très élevée et exempte de polymères en partant du surnageant IV, et notamment IV-4, ou de la fraction V (mise en solution) de COHN, ou d'un surnageant ou fraction plasmatique de composition analogue provenant d'un fractionnement alcoolique ou non, tout en maintenant à une valeur basse les coûts de l'installation et de sa mise en oeuvre.

L'invention a pour objet un procédé pour isoler l'albumine à partir des surnageants ou fractions ci-dessus, comprenant deux étapes de chromatographie par échange d'ions, caractérisé en ce que les seules étapes de chromatographie par échange d'ions sont une étape sur colonne échangeuse d'anions hydrophile avec fixation de l'albumine sur la colonne, puis élution, et une étape sur colonne échangeuse d'anions hydrophobe. Les échangeurs d'anions peuvent être indifféremment faibles ou forts.

Les étapes du procédé sont effectuées de préférence dans cet ordre, mais peuvent également l'être dans l'ordre inverse moyennant une augmentation du volume de l'échangeur d'anions hydrophobe.

Ce procédé permet d'obtenir une albumine de pureté très élevée, supérieure à 99 % en électrophorèse sur acétate de cellulose, exempte d'impuretés détectées en immunoélectrophorèse croisée et très stable puisque exempte de quantité significative de polymères (<1% après pasteurisation légale 10 h à 60°C). Le rendement de la purification chromatographique est supérieur à 90 % par rapport aux protéines à traiter.

De préférence, la colonne échangeuse d'anions hydrophile est une colonne de support chromatographique DEAE-SPHERODEX® (billes de silice poreuses revêtues d'un polymère hydrophile dextrane portant des groupements amine tertiaire DEAE ; échangeur d'anions faible, hydrophile) et la colonne échangeuse d'anions hydrophobe est une colonne de support chromatographique QMA-SPHEROSIL® (billes de silice poreuses revêtues d'un polymère hydrophobe polyvinyl toluène portant des groupements ammonium quaternaire QMA ; échangeur d'anions fort, hydrophobe).

Plus généralement, on peut utiliser des supports chromatographiques à base de silice poreuse revêtue d'un polymère soit hydrophile (par exemple dérivé du dextrane), soit hydrophobe (par exemple dérivé de polyvinyl toluène).

On peut également utiliser des supports équivalents aux supports ci-dessus, notamment :
a) supports à base de silice minérale poreuse revêtue d'autres polymères :
   - polymères hydrophiles : agarose, polyvinyles hydrophiles, polyacrylamides hydrophiles ou polymères équivalents non toxiques et non dénaturants pour les protéines.
   - polymères hydrophobes : les mêmes que ci-dessus mais rendus hydrophobes par greffage ou coréticulation de groupements hydrophobes tels que des groupements styrène, hexyle et d'une façon générale chaînes aliphatiques et groupements aromatiques procurant un caractère hydrophobe, étant entendu que les polymères doivent être non toxiques et non dénaturants pour les protéines.
b) supports à base de polymères organiques hydrophiles ou rendus hydrophobes comme ci-dessus:
   - polysaccharides (cellulose, dextrane, agarose, etc.)
   - polyvinyle
   - polyacrylamide
   - mixte polyacrylamide-agarose
   - ou équivalents non toxiques et non dénaturants pour les protéines.

Les polymères ci-dessus doivent porter des groupements chimiques leur conférant les propriétés d'échange d'anions selon l'utilisation prévue.

La présente invention a également pour objet une albumine obtenue selon le procédé conforme à l'invention.

L'invention va être maintenant décrite plus en détail à l'aide d'exemples de réalisation de l'invention.

### Exemple 1.

6 kg de la fraction V de COHN, correspondant à 61,4 litres de plasma citraté à environ 50 g/l de protéines totales sont dissous dans de l'eau déminéralisée apyrogène à +4°C. La solution est ajustée à pH 5,25 et résistivité de 1500 Ωcm. Le taux de protéines est de 23,9 g/l. La solution est filtrée à température ambiante sur une colonne de DEAE-SPHERODEX® de 9 kg de support prééquilibré en tampon phosphate de sodium 10 mM pH 5,25. Après rinçage de la colonne dans le même tampon, l'albumine est éluée par une solution tampon d'acétate de sodium 25 mM pH 4,7. On obtient 61,3 litres de solution d'albumine à 21,1 g/l de protéines. Cette solution est ensuite filtrée à température ambiante sur une colonne de 4 kg de QMA-SPHEROSIL® prééquilibrée dans une solution tampon acétate de sodium 25mM pH 4,7. Le filtrat est ajusté à pH 6,5, est diafiltré sur des membranes d'ultrafiltre de seuil de coupure 10 kD contre NaCl 6 g/l pour éliminer l'acétate de sodium et ensuite concentré à plus de 200 g/l de protéines sur le même ultrafiltre. La solution est ajustée ensuite en protéines à 200 g/l et en stabilisants : caprylate de sodium 2,7 g/l, N-acétyl-tryptophane 4 g/l, sodium 145 meq/l, Tween 80 15 mg/l et pH 6,95. Elle est ensuite pasteurisée 10 h à 60°C et soumise aux contrôles finaux. Le rendement global est de 21,7 g d'albumine purifiée par litre de plasma Initial. Il est de 91 % des protéines de la fraction V. La pureté en électrophorèse sur acétate de cellulose est de 100 % et le taux de polymères est de 0 %. Aucune impureté n'est détectable en analyse par immunoélectrophorèse croisée.

### Exemple 2.

142,5 litres de filtrat IV 4 de COHN, correspondant à 70 litres de plasma citraté à environ 50 g/l de protéines totales, sont dilués au 1/2 dans de l'eau déminéralisée apyrogène à +4°C. Cette solution est diafiltrée à pH 5,5 contre de l'eau déminéralisée apyrogène de manière à obtenir une résistivité de 1500 Ωcm pour un pH ajusté à 5,25. La solution est ensuite traitée comme dans l'exemple 1 sur une colonne de 9 kg de DEAE-SPHERODEX® et une colonne de 4 kg de QMA-SPHEROSIL®, puis analysée après pasteurisation comme précédemment.

Le rendement final est de 24,3 g d'albumine purifiée par litre de plasma initial. Il est de 94 % des protéines du surnageant IV.4. La pureté en électrophorèse sur acétate de cellulose est de 100 % et le taux de polymères est de 0 %. Aucune impureté n'est détectée en immunoélectrophorèse croisée.

La quantité de support chromatographique utilisée dans cet exemple est de 3,2 kg de DEAE-SPHERODEX® pour 25 l de plasma et de 1,4 kg de QMA-SPHEROSIL® pour 25 l de plasma. Le procédé de l'invention n'augmente pas les quantités de support chromatographique par rapport aux procédés antérieurs partant d'un surnageant ou d'une fraction de COHN et, par rapport au procédé antérieur de la demande de brevet français FR-A-2 543 448, il est réalisé une économie importante de support chromatographique, du fait, en particulier, de la suppression de l'échangeur de cations, et, par suite, des quantités de tampons et éluants nécessaires, tout en conduisant à une albumine de pureté comparable sur le plan électrophorétique.

Le procédé selon l'invention permet aussi de diminuer les coûts d'investissement et de maintenance liés au nombre d'étapes de chromatographie, donc au nombre de colonnes, par rapport à la demande FR-A-2 543 448 tout en procurant un produit de qualité au moins égale.

Du fait de sa haute pureté, outre l'utilisation clinique, cette albumine peut être utilisée comme agent adjuvant ou stabilisant de produits biologiques de haute pureté à haute activité spécifique, comme les facteurs de coagulation hautement purifiés. En particulier, elle peut être utilisée comme composant de milieux de culture définis, sans sérum animal, pour la production de molécules recombinantes à activité thérapeutique ou comme excipient dans les formulations pharmaceutiques finales de ces molécules.

## Revendications

1. Procédé pour isoler l'albumine à partir du surnageant IV, et notamment IV-4, ou de la fraction V de COHN ou d'un surnageant ou fraction plasmatique de composition analogue provenant d'un fractionnement alcoolique ou non, comprenant deux étapes de chromatographie par échange d'ions, caractérisé en ce que les seules étapes de chromatographie par échange d'ions sont une étape sur colonne échangeuse d'anions hydrophile avec fixation de l'albumine sur la colonne, puis élution, et une étape sur colonne échangeuse d'anions hydrophobe.

2. Procédé selon la revendication 1, caractérisé en ce que l'on traite le surnageant IV et notamment IV-4, ou la fraction V de Cohn ou un surnageant ou fraction plasmatique de composition analogue provenant d'un fractionnement alcoolique.

3. Procédé selon la revendication 2, caractérisé en ce que l'on fait passer le surnageant IV-4 ou la fraction V de COHN dans une colonne chromatographique contenant des billes de silice poreuses revêtues d'un polymère hydrophile, puis dans une colonne chromatographique contenant des billes de silice poreuses revêtues d'un polymère hydrophobe.

4. Procédé selon la revendication 3, caractérisé en ce que le polymère hydrophile est un dérivé du dextrane et le polymère hydrophobe est un dérivé du polyvinyl toluène.

5. Procédé selon la revendication 4, caractérisé en ce que le polymère hydrophile est un dextrane portant des groupements amine tertiaire et le polymère hydrophobe est un polyvinyl toluène portant des groupements ammonium quaternaire.

6. Procédé selon la revendication 3, caractérisé en ce que le polymère hydrophile est choisi parmi : agarose, polyvinyles hydrophiles, polyacrylamides hydrophiles.

7. Procédé selon la revendication 3, caractérisé en ce que le polymère hydrophobe est choisi parmi : agarose, polyvinyles ou polyacrylamides rendus hydrophobes par greffage ou coréticulation de groupements hydrophobes.

8. Procédé selon la revendication 2, caractérisé en ce que l'on fait passer le surnageant IV-4 ou la fraction V de COHN dans des colonnes contenant un polymère organique hydrophile ou hydrophobe.

9. Procédé selon la revendication 8, caractérisé en ce que le polymère est choisi parmi : polysaccharides, polyvinyles, polyacrylamides, mixte polyacrylamide-agarose.

## Claims

1. Method for isolating albumin from the supernatant IV, and in particular IV-4, or from the fraction V of COHN or from a supernatant or plasma fraction of similar composition coming from an alcoholic or non-alcoholic fractionation, comprising two stages of chromotography by ion exchange, characterised in that the only stages of chromotography by ion exchange are a stage on a hydrophilic anion exchange column with fixing of the albumin on the column, then elution, and a stage on a hydrophobic anion exchange column.

2. Method according to Claim 1, characterised in that the supernatant IV and in particular IV-4, or the fraction V of COHN or a supernatant or plasma fraction of similar composition coming from an alcoholic fractionation is treated.

3. Method according to Claim 2, characterised in that the supernatant IV-4 or the fraction V of COHN is made to pass through a chromotography column containing porous silica balls coated with a hydrophilic polymer, then through a chromotography column containing porous silica balls coated with a hydrophobic polymer.

4. Method according to Claim 3, characterised in that the hydrophilic polymer is a derivative of dextrane and the hydrophobic polymer is a derivative of polyvinyltoluene.

5. Method according to Claim 4, characterised in that the hydrophilic polymer is a dextrane comprising tertiary amine groups and the hydrophobic polymer is a polyvinyltoluene comprising quaternary ammonium groups.

6. Method according to Claim 3, characterised in that the hydrophilic polymer is chosen from "agarose, hydrophilic polyvinyls, hydrophilic polyacrylamides".

7. Method according to Claim 3, characterised in that the hydrophobic polymer is chosen from: agarose, polyvinyls or polyacrylamides made hydrophobic by grafting or co-reticulation of hydrophobic groups.

8. Method according to Claim 2, characterised in that the supernatant IV-4 or the fraction V of COHN is made to pass through columns containing a hydrophilic or hydrophobic organic polymer.

9. Method according to Claim 8, characterised in that the polymer is chosen from: polysaccharides, polyvinyls, polyacrylamides, mixed polyacrylamide-agarose.

## Patentansprüche

1. Verfahren zum Isolieren von Albumin ausgehend von der Fraktion IV und insbesondere IV-4 oder der Fraktion V nach Cohn oder von einem überstehenden Anteil bzw. einer Plasma-Fraktion analoger Zusammensetzung, die von einer alkoholischen oder nicht-alkoholischen Fraktionierung stammt, das zwei Ionenaustauschchromatographie-Stufen umfaßt, dadurch gekennzeichnet, daß die einzigen Ionenaustauschchromatographie-Stufen eine Stufe an einer hydrophilen Anionenaustauscher-Säule mit Fixierung des Albumins an der Säule und anschließendem Eluieren und eine Stufe an einer hydrophoben Anionenaustauscher-Säule sind.

2. Verfahren nach Anspruch 1, dadurch gekennzeichnet, daß man die Fraktion IV und insbesondere IV-4 oder die Fraktion V nach Cohn oder einen überstehende Anteil bzw. eine Plasma-Fraktion analoger Zusammensetzung, die von einer alkoholischen Fraktionierung stammt, behandelt.

3. Verfahren nach Anspruch 2, dadurch gekennzeichnet, daß man die Fraktion IV-4 oder die Fraktion V nach Cohn durch eine Chromatographie-Säule, die poröse Siliziumdioxid-Kügelchen, beschichtet mit einem hydrophilen Polymeren enthält und anschließend durch eine Chromatographie-Säule, die poröse Siliziumdioxid-Kügelchen, die mit einem hydrophoben Polymeren beschichtet sind enthält, passieren läßt.

4. Verfahren nach Anspruch 3, dadurch gekennzeichnet, daß das hydrophile Polymere ein Dextran-Derivat und das hydrophobe Polymere ein Polyvinyltoluol-Derivat ist.

5. Verfahren nach Anspruch 4, dadurch gekennzeichnet, daß das hydrophile Polymere ein Dextran ist, das tertiäre Aminogruppen trägt und das hydrophobe Polymere ein Polyvinyltoluol ist, das quarternäre Ammoniumgruppen trägt.

6. Verfahren nach Anspruch 3, dadurch gekennzeichnet, daß das hydrophile Polymere ausgewählt wird aus: Agarose, hydrophilen Polyvinylen, hydrophilen Polyacrylamiden.

7. Verfahren nach Anspruch 3, dadurch gekennzeichnet, daß das hydrophobe Polymere ausgewählt wird aus: Agarose, Polyvinylen oder Polyacrylamiden, die durch Pfropfen oder Vernetzung mit hydrophoben Gruppen hydrophob gemacht wurden.

8. Verfahren nach Anspruch 2, dadurch gekennzeichnet, daß man die Fraktion IV-4 oder die Fraktion V nach Cohn durch Säulen passieren läßt, die ein hydrophiles oder hydrophobes organisches Polymeres enthalten.

9. Verfahren nach Anspruch 8, dadurch gekennzeichnet, daß das Polymere ausgewählt wird aus: Polysacchariden, Polyvinylen, Polyacrylamiden, Polyacrylamid-Agarose-Gemisch.
